Europäisches Patentamt

European Patent Office

Office européen des brevets

(19)

(11) Numéro de publication: **0 034 516**
**A1**

(12) ## DEMANDE DE BREVET EUROPEEN

(21) Numéro de dépôt: **81400138.4**

(22) Date de dépôt: **29.01.81**

(51) Int. Cl.³: **A 61 B 17/38**
**A 61 N 1/06, A 61 N 1/40**
**A 61 N 1/08**

(30) Priorité: **08.02.80 FR 8002752**

(43) Date de publication de la demande:
**26.08.81 Bulletin 81/34**

(84) Etats contractants désignés:
**AT BE CH DE GB IT LI LU NL SE**

(71) Demandeur: **SOCIETE ANONYME DE**
**TELECOMMUNICATIONS**
**40 avenue de New York**
**F-75116 Paris(FR)**

(72) Inventeur: **Chable, Yves Antonin**
**29, rue de Coetquen**
**F-22100 Dinan(FR)**

(72) Inventeur: **Lacroix, Serge**
**23 boulevard Flaud**
**F-22100 Dinan(FR)**

(72) Inventeur: **Rault, Philippe Maurice**
**Le Jardin des Ecouères Bourg de Lehon**
**F-22100 Dinan(FR)**

(74) Mandataire: **Bloch, Robert et al,**
**Cabinet ROBERT BLOCH 39 avenue de Friedland**
**F-75008 Paris(FR)**

(54) **Dispositif de traitement hyperthermique par champ électrique à une fréquence radio.**

(57) L'invention a pour objet un dispositif de traitement théra-peutique par hyperthermie comprenant und générateur de puissance (2) à fréquence radio, au moins deux applicateurs (7, 8) de cette puissance au corps du patient et un ensemble de mesures des températures en plusieurs points de la zone traitée.

Dans le dispositif de l'invention, les applicateurs (7,8) sont réalisés dans des matériaux souples et déformables absorbant l'humidité et la face desdits applicateurs en contact avec la peau du patient est conductrice de l'électricité, reliée électriquement aux sorties de puissance (4) dudit générateur (2) et en bon contact électrique avec la peau du patient.

FIG.1

Dispositif de traitement hyperthermique par champ électrique à une fréquence radio.

Le dispositif objet de la présente invention fait partie du matériel à utilisation thérapeutique.

La présente invention a pour objet un dispositif de traitement médical de certaines maladies humaines, tel que les cancers.

Plus particulièrement, il s'agit d'un dispositif de traitement local ou loco-régional des cancers, en particulier des cancers profonds localisés, traitement réalisé par une surchauffe de la masse tumorale, obtenue sans affecter les tissus sains environnants.

Ce traitement hyperthermique est obtenu, de façon connue, par l'action d'un générateur de puissance à fréquence radio, par exemple à 13,56 MHz, laquelle puissance est appliquée au moyen d'applicateurs ou électrodes à la zone à traiter du corps du patient, tandis que la température de ladite zone est contrôlée. Cette zone comprend la masse tumorale dont on cherche à élever la température au maximum, pour obtenir la nécrose de ladite masse, et les tissus sains environnants dont la température ne doit pas dépasser 43°C.

Les différentes températures obtenues en différents points de la zone traitées sont le résultat de trois paramètres ; la puissance électrique appliquée, la nature des tissus et l'irrigation sanguine desdits tissus. L'action de ces trois paramètres permet d'atteindre un équilibre dynamique régi par des lois simples :
- les points les plus chauffés sont aussi les plus refroidis si l'irrigation par le sang est suffisante,
- les tissus sains sont bien chauffés et leur température est régulée, au-dessous de la température létale, par le débit sanguin,

- les os, et les tissus adipeux reçoivent peu de puissance, mais cependant ils risquent d'être surchauffés car ils sont mal irrigués dans leur masse,

- les tumeurs cancéreuses reçoivent beaucoup de puissance car elles sont bien hydratées, et, étant mal irriguées elles sont rapidement surchauffées, les points les plus chauds étant situés au centre des tumeurs.

Des dispositifs pour la mise en oeuvre d'un tel traitement par hyperthermie sont déjà connus, en particulier, par l'article "Tumor Eradication in the Rabbit by Radiofrequency Heating" publié par J.A. Dickson S.A. Shab, D. Waggott and W.B. Whalley dans la revue Cancer Research vol. 37 july 1977 p. 2162-2169. Toutefois le dispositif décrit dans cet article n'est pas adapté pour les traitements humains, en particulier parce qu'il utilise pour appliquer la tension électrique de traitement au coprs du patient des applicateurs rigides qui risquent de meurtrir le patient, s'ils sont appliqués avec une forte pression, ou de provoquer des brûlures, s'ils ne sont pas parfaitement appliqués sur la peau du patient.

Fait également partie de l'art antérieur, un dispositif de traitement hyperthermique connu sous la marque déposée "Magnétrode". Ce dispositif fabriqué et distribué par HYPERTHERMIA Division of Henry Electronics, Inc utilise de préférence le champ magnétique créé par un solénoïde ou un tore entourant la zone à traiter ou placé au voisinage de la surface à traiter.

Tous ces dispositifs connus, de même que le dispositif selon l'invention, utilisent un générateur de radiofréquences à 13,56 MHz, toutefois le dispositif selon l'invention se distingue des dispositifs connus essentiellement par la nature de ses applicateurs, par le système particulier de mesure des températures dans la zone soumise au traitement et par la façon particulière dont est réalisé l'accord du générateur de puissance en fonction de l'impédance de charge effectivement mise en oeuvre.

Pour cela, le dispositif selon l'invention, qui se compose d'un générateur de puissance à fréquence radio, d'au moins deux applicateurs de cette puissance au corps du patient, d'un ensemble de mesures de températures en plusieurs points de la zone traitée, est caractérisé en ce que les applicateurs sont réalisés avec des matériaux souples et déformables, qu'ils absorbent l'humidité, qu'ils sont conducteurs électriques, reliés électriquement aux sorties de puissance dudit générateur et en bon contact électrique avec la peau du patient.

A titre d'exemple non limitatif, lesdits applicateurs se composent essentiellement d'un tissu conducteur électrique souple dont l'une des faces, ou surface efficace, imprégnée d'un gel conducteur électrique, est en contact direct avec la peau du patient dont l'autre face est en contact avec la première face d'un tricot métallique conducteur sur l'autre face duquel, d'une part sont soudés individuellement, et selon une répartition a peu près uniforme, chaque brin d'un conducteur multibrins souple gainé d'un plastique isolant dans sa partie non en contact avec ledit tricot, et , d'autre part, est appliquée une plaque de mousse élastique de densité moyenne recouverte elle-même d'une couche protectrice souple sur laquelle sont appliquées, en partie, une ou plusieurs bandes élastiques adhésives dont les autres parties adhèrent à la peau du patient.

Selon une variante de réalisation de la présente invention, ladite plaque de mousse élastique peut être remplacée par un ballonnet gonflable. Dans ce cas ledit ballonnet gonflable qui assure la pression nécessaire à l'application du tissu conducteur souple sur la peau du patient, est lui-même entouré de compresses absorbantes destinées à absorber l'humidité.

Toujours selon la présente invention lesdits applicateurs ont une surface efficace, qui est celle du tissu conducteur souple, adaptée à la zone à traiter, le tricot métallique conducteur souple de surface plus réduite est centré sur ledit tissu conducteur souple et la plaque de mousse, de surface au moins identique au tissu conducteur souple, recouvre l'ensemble du tissu conducteur et du tricot métallique conducteur.

Le dispositif de traitement thérapeutique par hyperthermie selon l'invention qui comporte, entre autres choses, un ensemble de mesures de températures en plusieurs points de la zone traitée comprenant essentiellement $n$ thermocouples placés chacun à l'intérieur d'une aiguille hypodermique est également caractérisé en ce que sur chacun des deux fils de chaque thermocouple est inséré un filtre passe-bas ou coupe bande le plus près possible de la sortie dudit thermocouple du corps du patient, que la sortie de chacun de ces filtres est reliée à l'entrée d'un dispositif de commutation et de comparaison des températures et en ce la sortie de ce dispositif de comparaison et de commutation est reliée à travers un filtre passe-bas à l'entrée d'un enregistreur essentiellement à vibreur et amplificateur alternatif.

Toujours selon la présente invention, le dispositif de commutation et de comparaison des températures comprend essentiellement un commutateur et une plaquette de $2(n+1)$ points de raccordements isothermes, chaque point de raccordement étant relié, d'une part, respectivement à l'un des 2 fils des $n$ thermocouples ou à l'un des 2 fils d'un thermocouple de référence et, d'autre part, pour les $2n+1$ premiers points de raccordement aux bornes d'entrée du commutateur rotatif et pour le point de raccordement $2n+2$ directement à la deuxième borne de sortie du dispositif de commutation et de comparaison dont la première borne de sortie est reliée à la borne de sortie du commutateur.

Toujours selon l'invention, ledit thermocouple de référende est un thermocouple de même nature que les thermocouples de mesure et il est placé en permanence dans un bain thermostatique composé d'un mélange d'eau distillée et de glace pure pilée dont la température est contrôlée par un ou plusieurs thermomètres de précision.

Le dispositif selon l'invention peut être encore caractérisé en ce qu'il comporte à proximité immédiate des applicateurs, un circuit d'accord permettant d'adapter la puissance directe émise par ledit générateur en fonction de la disposition des applicateurs de puissance de façon à obtenir une puissance réfléchie pratiquement nulle.

Toujours selon l'invention, ledit dispositif comporte au moins deux applicateurs dont l'un est relié à un potentiel alternatif et l'autre à un potentiel nul, ou les deux à un potentiel alternatif.

Dans une variante du dispositif selon l'invention comportant trois applicateurs, l'un est relié à un potentiel alternatif et les deux autres à un potentiel nul, ou vice-versa.

Dans une autre variante du dispositif selon l'invention, les applicateurs reliés à un potentiel alternatif comporteront avantageusement une zone centrale conductrice reliée audit potentiel alternatif, une zone isolante entourant ladite zone centrale et une zone externe conductrice reliée ou non à un potentiel et entourant ladite zone isolante.

Selon une autre variante de la présente invention le générateur de puissance comporte p tubes de puissance alimentant p sorties déphasées de $\frac{2\pi}{p}$ reliées à, au moins, 2 p applicateurs de puissance.

Selon cette dernière variante, lesdits 2 p applicateurs seront avantageusement placés sur le corps du patient de telle sorte que les p champs électriques ainsi créés contiennent tous la zone à traiter.

Dans tous les cas de réalisation de la présente invention il est prévu que la tension distribuée sur les applicateurs ne comporte pas de composante continue et ce, grâce à une double sécurité. A titre d'exemple, deux condensateurs l'un de 100 pF et l'autre de 220 pF sont mis en série sur le circuit de sortie du générateur de puissance.

Le principal avantage du dispositif thérapeutique selon l'invention réside dans la nature souple de ses applicateurs par ailleurs parfaitement conducteurs électriques ce qui permet un contact électrique direct avec la peau du patient et évite ainsi tout risque de brûlure dudit patient.

Un autre avantage du dispositif thérapeutique selon l'invention réside dans la possibilité de choisir le nombre, la forme et les dimensions des applicateurs en fonction de chaque cas à traiter.

Un autre avantage du dispositif thérapeutique selon l'invention réside dans la grande précision du dispositif de mesure des températures en différents points de la zone traitée et ceci en cours de traitement, le dit dispositif de mesure des températures étant rendu selon l'invention insensible au champ des ondes radiofréquences émises par le générateur de puissance et par tout le circuit électrique qui lui est connecté.

Un autre avantage du dispositif thérapeutique selon l'invention réside dans la possibilité d'accord réglable de l'impédance de charge du générateur de puissance ; de cet accord réglable résulte une meilleure utilisation de la puissance émise et une réduction du champ parasite créé.

D'autres caractéristiques et d'autres avantages du dispositif thérapeutique selon l'invention apparaitront au cours de la description qui suit pour la compréhension de laquelle on se reportera aux figures données en annexe.

- La figure 1 est une vue d'ensemble schématisée du dispositif thérapeutique selon l'invention.
- La figure 2 représente la vue en coupe d'un des applicateurs selon l'invention.
- La figure 3 représente un exemple de réalisation du filtre placé sur chaque fil de thermocouple près du corps du patient.
- La figure 4 représente schématiquement le dispositif de commutation et de comparaison des températures.
- La figure 5 représente le schéma électrique du dispositif d'accord.
- La figure 6 représente un exemple de positionnement de trois applicateurs.
- La figure 7-a et la figure 7-b représentent un exemple de forme de deux applicateurs.
- La figure 8 représente un exemple d'utilisation d'un dispositif selon l'invention utilisant un générateur à trois sorties déphasées.
- La figure 9 représente le schéma électrique du filtre utilisé à l'entrée de l'enregistreur de température.

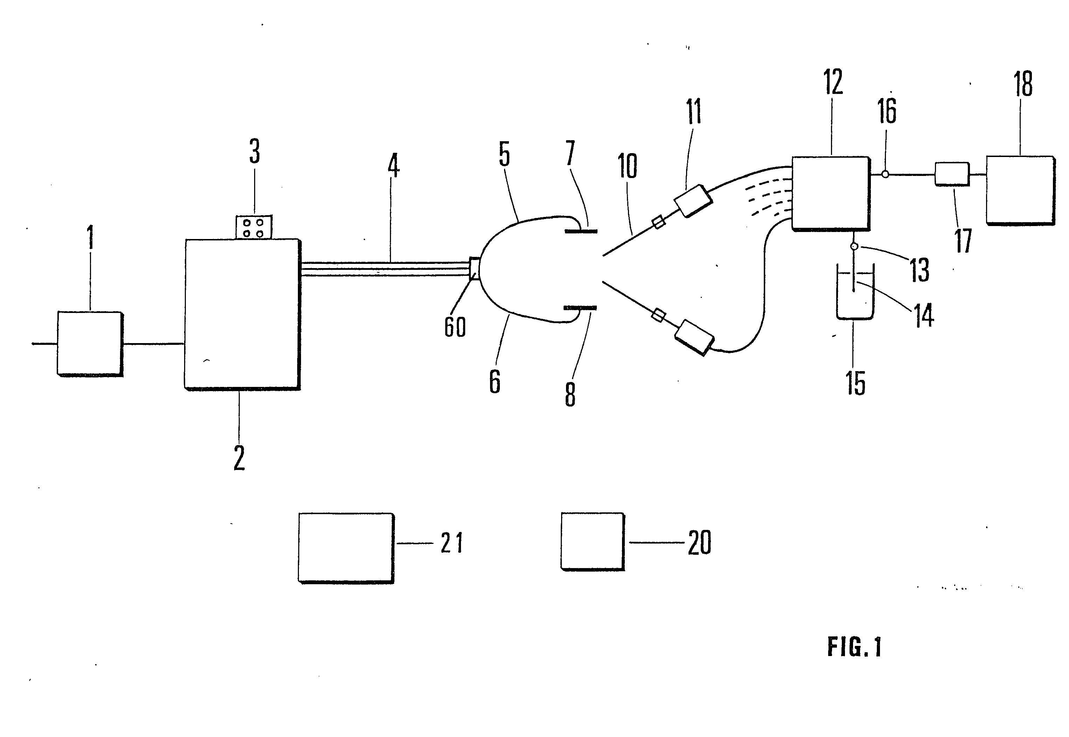

L'ensemble du dispositif selon l'invention représenté sur la figure 1 comporte essentiellement un régulateur de tension 1, un générateur de puissance 2 et son tableau de commande 3, une ligne de sortie de puissance 4 qui se termine en 5 et 6 par les deux fils d'alimentation des applicateurs souples 7 et 8.

Fait également partie du dispositif selon l'invention la chaîne de mesure des températures qui se compose essentiellement de $n$ thermocouples, tel que 10, et de $n$ filtres passe-bas ou coupe-bande tel que 11, tous reliés à un commutateur de thermocouples 12 comportant n + 1 paires de bornes d'entrée dont la (n + 1)ième paire est reliée à un thermocouple 14 dit de référence plongé dans un bain de référence 15. La paire de borne de sortie 16 dudit commutateur de thermocouple 12 est reliée à l'entrée d'un filtre passe-bas 17 dont la sortie est reliée à l'entrée d'un dispositif enregistreur 18.

Ne font pas réellement partie du dispositif selon l'invention, mais sont nécessaires à sa bonne mise en oeuvre un bain thermostatique 20 et une charge étalon 21, par exemple de 50 $\Omega$. Avant la mise en oeuvre du dispositif thérapeutique selon l'invention, le bain thermostatique 20 sert à étalonner les thermocouples tels que 10, et la charge étalon 21 sert à prérégler le générateur de puissance 2 et à vérifier son bon fonctionnement.

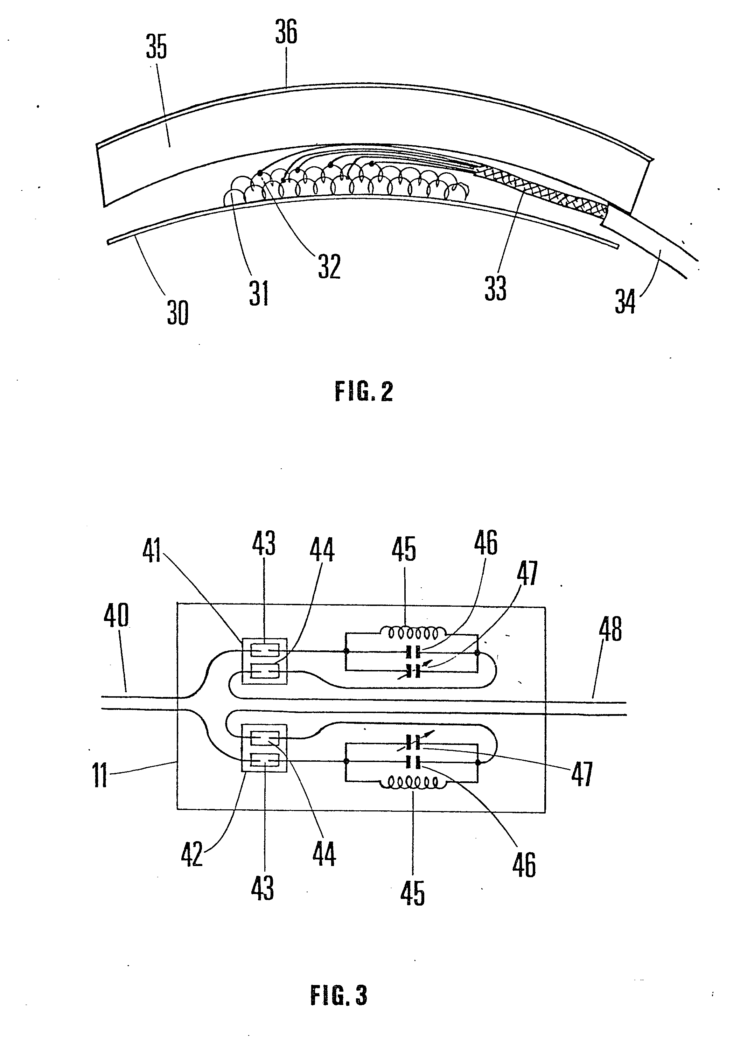

La figure 2 représente une vue en coupe transversale des applicateurs selon l'invention. Sur cette figure sont représentés, en 30, un tissu souple conducteur, de fils textiles absorbants et de fils métalliques très bons conducteurs, en 31, un tricot métallique conducteur sur lequel sont soudés individuellement, tel qu'en 32, les différents brins d'un conducteur souple 33 isolé, dans sa partie extérieure à l'applicateur proprement dit, par un revêtement plastique 34.

L'ensemble ainsi formé est recouvert par une plaque de mousse élastique moyenne densité 35, elle-même recouverte d'un revêtement souple 36, posé sur la face supérieure de ladite plaque 35.

Ce revêtement 36 lisse à sa partie supérieure permet la fixation aisée dudit applicateur sur le corps du patient au moyen de bandes élastiques adhésives médicales du type "Albuplast", adhérant par ailleurs à la peau du patient.

A titre d'exemple non limitatif, le tissu conducteur souple est un tissu utilisé habituellement pour la confection des vêtements des escrimeurs de compétition. Ce tissu est alors découpé à la forme exacte que l'on désire pour l'applicateur. Sa surface efficace est ensuite enduite d'un gel conducteur du type de celui utilisé lors de l'établissement d'électrocardiogrammes et appliqué fermement à l'endroit prévu. Ensuite est posé sur ce tissu conducteur souple, une, deux ou trois épaisseurs de tricot métallique sur les mailles duquel sont soudés individuellement et selon une répartition à peu près uniforme les brins du fil d'alimentation. Toujours à titre d'exemple, ledit fil est du câble THT 15 KV du type câble non antiparasité utilisé autrefois pour l'alimentation des bougies des automobiles.

En se reportant à la figure 1, les thermocouples, tel que 10, sont du type chromel - alumel. On trouve dans le commerce des fils très fins chromel - alumel qui sont déjà placés dans une gaine en acier inoxydable remplie avec de la poudre de magnésie ; il suffit alors de faire avec soin la soudure dudit thermocouple et de glisser celui-ci dans un cathéter ou aiguille hypodermique dont le diamètre extérieur est de l'ordre de 0,6 à 1 mm, de telle sorte que ladite soudure de thermocouple soit légèrement sortie dudit cathéter. Une radiographie des thermocouples permet de contrôler le bon positionnement de l'ensemble de façon à être sûr que le thermocouple mesure la température à l'extrémité de la sonde et non la température à l'intérieur de l'aiguille hypodermique.

Immédiatement à la sortie de l'aiguille hypodermique les fils chromel - alumel sont interrompus par un filtre, tel que 11, dont un exemple de réalisation détaillée est représenté par la figure 3.

On voit sur cette figure 3 que les deux fils du thermocouple 40 arrivent respectivement sur les plots 41 et 42. Chacun de ces plots comporte un connecteur d'arrivée 43 et un connecteur de départ 44 qui sont isothermes : pour
cèla ils sont montés sur une plaquette d'oxyde de béryllium qui est un bon conducteur thermique tout en étant
un isolant électrique. Entre ces deux connecteurs est inséré le filtre proprement dit qui est ici, à titre d'exemple, un circuit bouchon composé d'une self 45, d'un condensateur fixe 46 et d'un condensateur variable 47, mis
en parallèle. A titre d'exemple, la self 45 est une self
bobinée en argent d'une valeur de 2 $\mu H$, le condensateur 46
est un condensateur céramique de 68 pF et le condensateur
47 est un condensateur ajustable de 1 à 30 pF ; toutes les
connections entre les connecteurs d'arrivée 43 et les
connecteurs de départ 44 sont en fils d'argent et à la
sortie du connecteur de départ 44 on utilise de nouveau
des fils chromel - alumel 48.

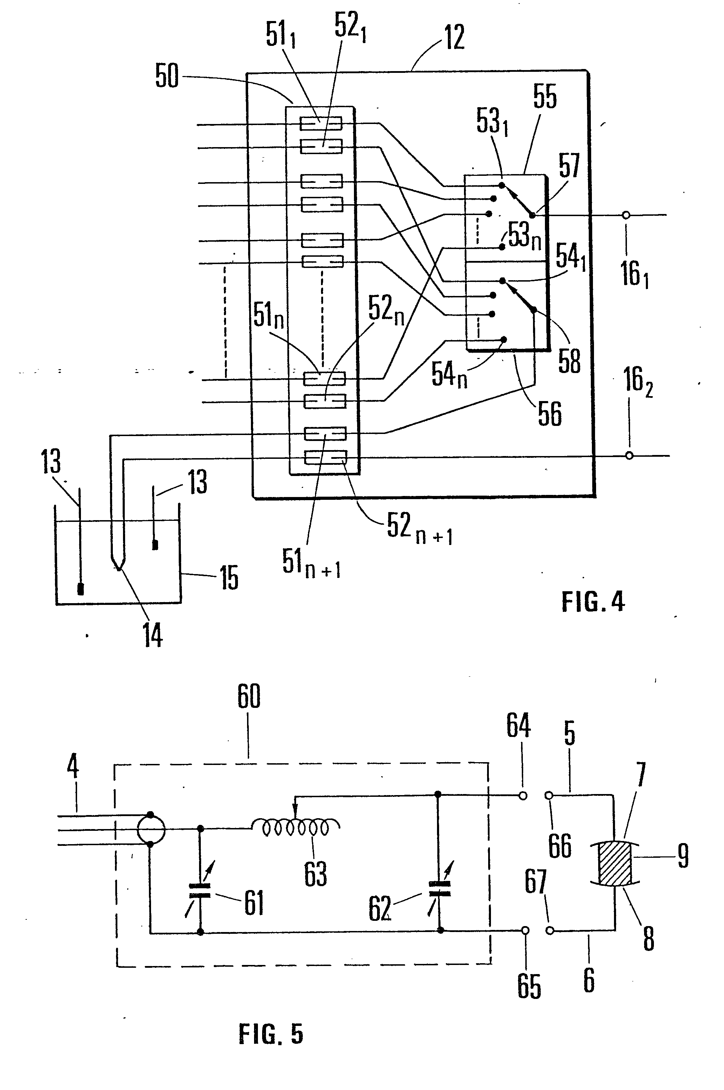

La figure 4 représente le commutateur 12 permettant, d'une
part, de sélectionner le thermocouple 10 dont l'on désire
enregistrer les variations de température et, d'autre
part, de relier ledit thermocouple au thermocouple de
référence 14.

Ce commutateur 12 comporte essentiellement une plaque
isotherme 50 sur laquelle sont fixées n + 1 paires, $51_1$ et
$52_1$ à $51_{n+1}$ et $52_{n+1}$, de plots de raccordement. A titre
d'exemple, la plaque isotherme est en cuivre et les plots
de raccordement sont en céramique. Lesdits plots de raccordement reçoivent à l'une de leurs extrémités les extrémités des 2 n fils chromel - alumel des n thermocouples
de mesure de température tel que 10 et les deux fils du
thermocouple de référence 14. Les sorties des plots de
raccordement $51_1$ à $51_n$ inclus sont reliées respectivement
aux différents plots d'entrée $53_1$ à $53_n$ d'un premier commutateur 55 jumelé avec un second commutateur 56 dont les
plots d'entrée $54_1$ à $54_n$ sont respectivement reliés aux
sorties des plots de raccordement $52_1$ à $52_n$. La borne de
sortie 58 du commutateur 56 est reliée à la borne de sortie du plot de raccordement $51_{n+1}$, tandis que la borne de sortie du plot de raccordement $52_{n+1}$ est reliée à la borne de sortie $16_2$ du dispositif 12, la borne de sortie $16_1$ dudit dispositif 12 étant reliée à la borne de sortie 57 du premier commutateur 55.

Le thermocouple de référence 14 plonge dans un bain 15 d'eau distillée et de glace pure pilée thermostaté à 0°C et dont la température est contrôlée au moyen de thermomètres étalons 13.

La figure 5 représente le schéma électrique du dispositif permettant d'accorder la puissance émise par le générateur selon l'impédance de la charge qui est incluse entre les applicateurs. Ce dispositif d'accord 60 est placé entre le générateur et les applicateurs, le plus près possible du patient de manière à ce que la distance dudit dispositif aux applicateurs soit la plus courte possible. A titre d'exemple non limitatif sur la figure 5, deux capacités 61 et 62 réglables de 30 à 300 pF et une self 63 ajustable de 0 à 1 µH sont utilisées pour la réalisation de ce filtre, les deux capacités sont montées en parallèle entre le conducteur central et le conducteur extérieur du câble coaxial 4 venant du générateur de puissance, et la self est montée en série avec le conducteur central. Les deux bornes de sortie 64 et 65 de ce dispositif d'accord 60 sont destinées à être reliées aux bornes d'accès 66 et 67 des conducteurs très courts 5 et 6 alimentant les deux applicateurs 7 et 8. Selon l'indépance de la charge 9, le réglage des condensateurs 61 et 62 et l'ajustage de la self 63 sont effectués de façon à utiliser la puissance maximum disponible ; pour cela, la puissance directe et la puissance réfléchie sont mesurées avec précision et le réglage est effectué de telle façon que la puissance réfléchie soit toujours inférieure à 5% de la puissance directe. Grâce à ce dispositif d'accord 60, toutes sortes de configurations d'applicateurs peuvent être mises en oeuvre ; différentes formes, surfaces, distances, positions sur le corps et positions relatives desdits applicateurs que peuvent nécessiter les traitements et l'ana- des patients, peuvent alors être utilisées.

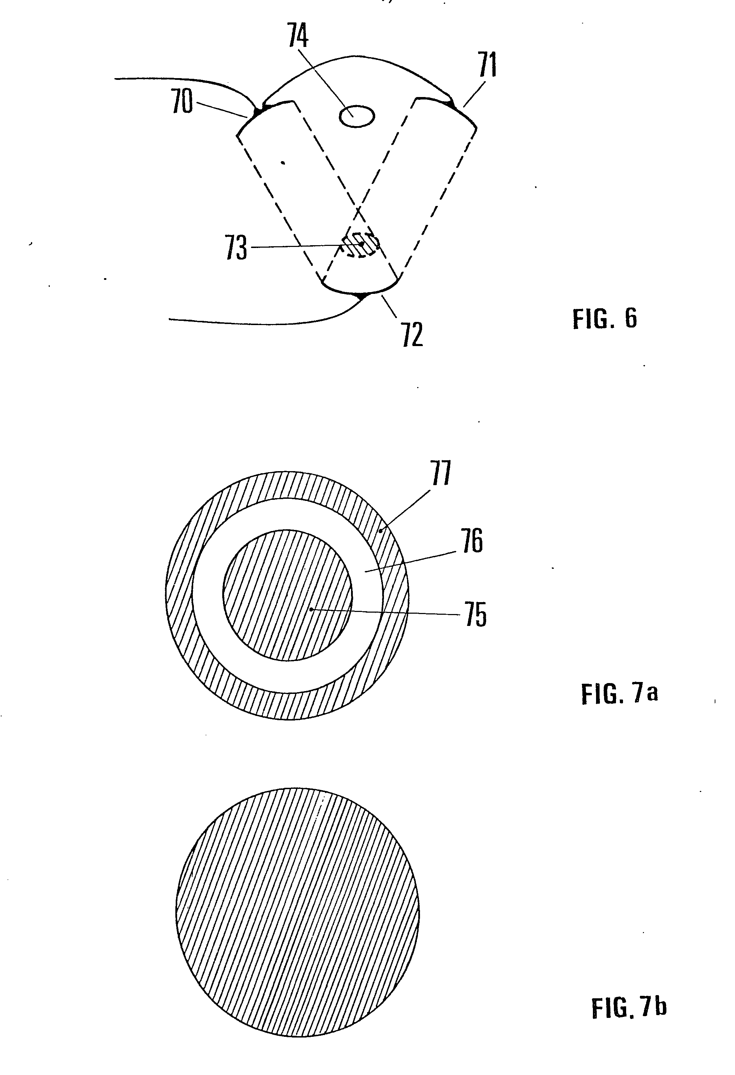

Ainsi selon l'invention, les électrodes souples peuvent être de forme variée et également en nombre variable. Ainsi sur la figure 5, deux applicateurs 7 et 8 sont placés en vis-à-vis, l'un 7 est porté à un potentiel alternatif et l'autre 8 est à la masse. Sur la figure 6, trois applicateurs sont utilisés, les deux applicateurs 70 et 71 étant à la masse et l'applicateur 72 étant à un potentiel alternatif. On obtient ainsi une bonne chauffe d'une tumeur sise par exemple en 73 alors qu'une zone fragile, par exemple osseuse sise en 74, n'est pas soumise au traitement hyperthermique.

De bons résultats ont été également obtenus en utilisant des applicateurs circulaires, dont l'un, représenté sur la figure 7-a présente une zone centrale 75 conductrice portée à un potentiel alternatif une zone 76 isolante et une zone 77 conductrice non reliée à un potentiel, laquelle sert en quelque sorte d'anneau de garde, et dont l'autre, représenté sur la figure 7-b, est entièrement conducteur et relié à la masse.

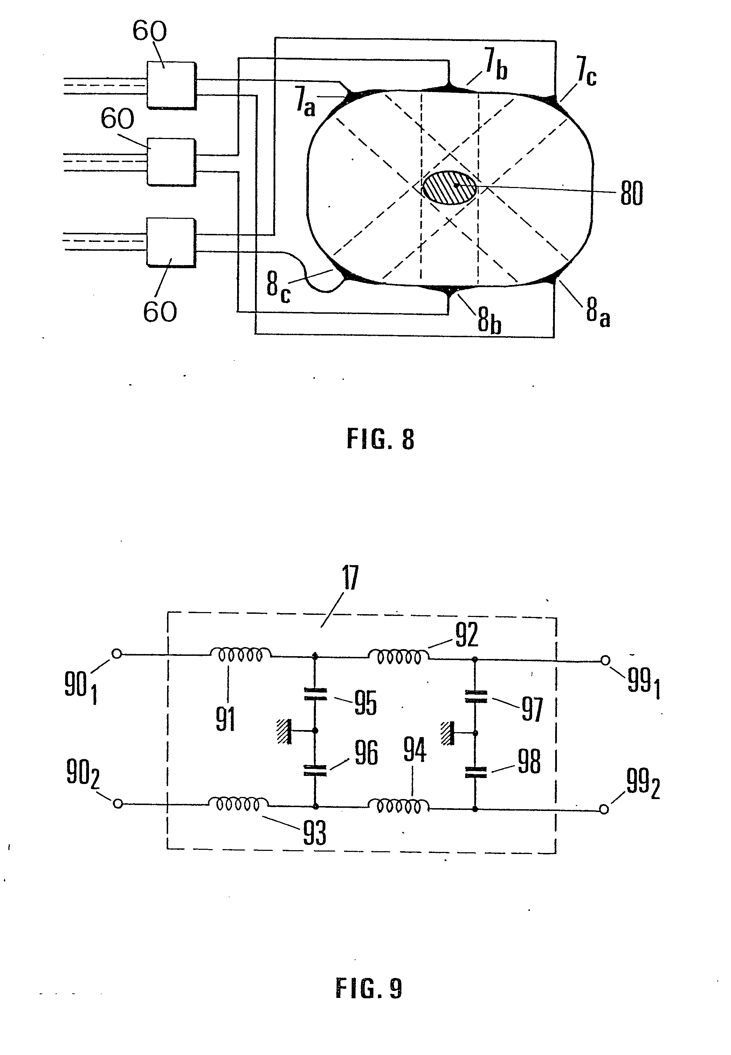

Toujours selon l'invention, il est également possible d'utiliser un générateur de puissance possèdant $\underline{p}$ tubes de puissance alimentant $\underline{p}$ sorties déphasées les unes par rapport aux autres de $\frac{\pi}{p}$ . A chacune des $\underline{p}$ sorties sont associés au moins deux applicateurs, la figure 8 représente à titre d'exemple, trois paires d'applicateurs 7a-8a, 7b-8b et 7c-8c alimentées par les trois sorties déphasées de $\frac{\pi}{3}$ d'un générateur de puissance ; chacune des sorties disposant d'un dispositif d'accord de puissance 60. La disposition des applicateurs est alors telle que la tumeur à soigner, sise par exemple en 80, se trouve traversée par les trois champs électriques.

Toujours selon l'invention, en se reportant à la figure 1, on voit qu'un filtre 17 est placé à l'entrée de l'enregistreur de température 18. A titre d'exemple non limitatif, la figure 9 représente le schéma électrique d'un filtre 17 dont l'utilisation permet une mesure et un enregistrement corrects de la température sans interrompre le fonctionnement du générateur de puissance. Les deux bornes d'entrée $90_1$ et $90_2$ dudit filtre 17 sont reliées respectivement aux bornes de sorties $16_1$ et $16_2$ du dispositif 12 de commutation de thermocouples. Toujours à titre d'exemple les selfs 91, 92, 93 et 94 ont une valeur de 47 µH et les condensateurs 95, 96, 97 et 98 sont des condensateurs céramiques de 56 nF. Les bornes de sortie $99_1$ et $99_2$ dudit filtre 17 sont reliées aux bornes d'entrée de l'enregistreur 18.

Ledit appareillage selon l'invention est destiné au traitement par hyperthermie des cancers localisés. Cet appareillage peut également être utilisé pour le traitement d'autres affections, par exemple pour les infections génito-urinaires résistantes aux antibiotiques.

0034516

14

Revendications.

1.- Dispositif de traitement thérapeutique par hyperthermie comprenant un générateur de puissance (2) à fréquence radio, au moins deux applicateurs (7,8) de cette puissance au corps du patient, un ensemble de mesures des températures en plusieurs points de la zone traitée, caractérisé par le fait, d'une part, que les applicateurs (7,8) sont réalisés dans des matériaux souples et déformables absorbant l'humidité, et d'autre part, par le fait que la face desdits applicateurs en contact avec la peau du patient est conductrice de l'électricité, reliée électriquement aux sorties de puissance (4) dudit générateur (2) et en bon contact électrique avec la peau du patient.

2.- Dispositif selon la revendication 1, dans lequel lesdits applicateurs se composent essentiellement d'un tissu souple 30, de fils textiles absorbants et de fils métalliques très bons conducteurs, dont l'une des faces, ou surface efficace, imprégnée d'un gel conducteur électrique, est en contact direct avec la peau du patient, dont l'autre face est en contact avec la première face d'un tricot métallique conducteur (31) sur l'autre face duquel, d'une part, sont soudés individuellement et selon une répartition à peu près uniforme, chaque brin (32) d'un conducteur multibrins souple (33) gainé d'un plastique isolant (34) dans sa partie non en contact avec ledit tricot, et d'autre part, est appliquée une plaque de mousse élastique (35) de densité moyenne recouverte elle-même d'une couche protectrice souple (36) sur laquelle sont appliquées en partie, une ou plusieurs bandes élastiques adhésives dont les autres parties adhérent à la peau du patient.

3.- Dispositif selon la revendication 2 dans lequel les applicateurs (7,8) ont une surface efficace, qui est celle du tissu conducteur souple (30), adaptée à la zone à traiter, le tricot métallique conducteur souple (31) de surface plus réduite est centré sur ledit tissu conducteur souple (30) et la plaque de mousse (35) de surface au moins identique au tissu conducteur souple (30) recouvre l'ensemble du tissu conducteur souple (30) et du tricot métallique conducteur (31).

4.- Dispositif de traitement thérapeutique selon la revendication 1, comportant, entre autres choses, un ensemble de mesures de températures en plusieurs points de la zone traitée comprenant essentiellement $n$ thermocouples (10) placés à l'intérieur d'aiguilles hypodermiques, caractérisé en ce que sur chacun des deux fils de chaque thermocouple (10) est inséré un filtre passe-bas ou coupe-bande (11), le plus près possible de la sortie dudit thermocouple (10) du corps du patient que la sortie de chacun de ces filtres (11) est relié à l'entrée d'un dispositif (12) de commutation et de comparaison des températures et en ce que la sortie de ce dispositif de comparaison et de commutation (12) est reliée à travers un filtre passe-bas (17) à l'entrée d'un enregistreur (18) essentiellement à vibreur et amplificateur alternatif.

5.- Dispositif selon la revendication 4, dans lequel le dispositif de comparaison et de commutation des températures (12) comprend essentiellement un commutateur (55) et une plaquette (50) de 2 (n+1) points de raccordement isothermes, chaque point de raccordement étant relié, d'une part, respectivement à l'un des 2 fils des $n$ thermocouples (10) ou à l'un des 2 fils d'un thermocouples de référence (14) et, d'autre part, pour les 2 n+1 premiers points de raccordement aux bornes d'entrée du commutateur (55) et pour le point de raccordement 2 n+2 directement à la deuxième borne de sortie ($16_2$) du dispositif de commutation et de comparaison (12) dont la première borne de sortie ($16_1$) est reliée à la borne de sortie du commutateur.

6.- Dispositif selon l'une des revendications 1 à 5, dans lequel un circuit d'accord (60) situé à proximité immédiate des applicateurs (7,8) permet d'adapter la puissance directe émise par le générateur de puissance (2) en fonction de la disposition desdits applicateurs (7,8) de façon à obtenir une puissance réfléchie pratiquement nulle.

7.- Dispositif thérapeutique selon la revendication 1, comportant deux applicateurs dont l'un est relié à un potentiel alternatif et l'autre à un potentiel nul.

8.- Dispositif thérapeutique selon la revendication 1, comportant deux applicateurs dont l'un est relié à un potentiel alternatif et dont l'autre est relié à un autre potentiel alternatif.

9.- Dispositif thérapeutique selon la revendication 1, comportant trois applicateurs dont l'un est relié à un potentiel alternatif et les deux autres à un potentiel nul ou alternatif ou vice-versa.

10.- Dispositif thérapeutique selon l'une des revendications 7 et 8, dans lequel le ou les applicateurs reliés à un potentiel alternatif ou nul comportent une zone conductrice centrale (75), effectivement reliée à ce potentiel alternatif ou nul et une zone conductrice annulaire (77) reliée ou non à un potentiel alternatif ou nul et séparée de la zone conductrice centrale par une zone isolante (76).

11.- Dispositif thérapeutique selon la revendication 1, caractérisé en ce que le générateur de puissance comporte $p$ tubes de puissance alimentant $p$ sorties déphasées de $\frac{\pi}{p}$ reliées à, au moins, 2 $p$ applicateurs de puissance.

0034516

FIG.1

35 36

30 31 32 33 34

**FIG. 2**

41 43 44 45 46 47

40 48

11

42 44 47

43 45 46

**FIG. 3**

FIG. 4

FIG. 5

74

70 71

73

72

**FIG. 6**

77

76

75

**FIG. 7a**

**FIG. 7b**

FIG. 8

FIG. 9

**Office européen des brevets**

## RAPPORT DE RECHERCHE EUROPEENNE

| CLASSEMENT DE LA DEMANDE (Int. Cl.³) |
|---|

### DOCUMENTS CONSIDERES COMME PERTINENTS

| Catégorie | Citation du document avec indication, en cas de besoin, des parties pertinentes | Revendica-tion concernée |
|---|---|---|
| | FR - A - 732 175 (HUTH)<br>* page 3, lignes 7 à 50; figure 1 * | 1-3 |
| | -- | |
| | FR - A - 915 335 (ROBINOT)<br>* page 1, ligne 60 à page 2, ligne 22; figure 1 * | 1 |
| | -- | |
| | US - A - 2 198 073 (BAYLESS)<br>* page 2, lignes 61 à 75 * | 1,2,8 |
| | -- | |
| | FR - A - 2 421 628 (CGR-MeV)<br>* page 3, ligne 12 à page 4, ligne 26; figures 1,2; page 5, ligne 23 à page 6, ligne 6; figure 7; page 7, ligne 32 à page 8, ligne 2 * | 4-6 |
| | -- | |
| | FR - A - 2 365 911 (LEVEEN)<br>* page 4, ligne 8 à page 5, ligne 13; figures 1,2 * | 9 |
| | -- | |
| | FR - A - 2 414 812 (DOLLEY)<br>* page 6, lignes 8 à 17; figure 4 * | 10 |
| | -- | |
| A | FR - A - 438 188 (SIEMENS)<br>* page 1, ligne 32 à page 2, ligne 14 * | 4 |
| | --                  ./.. | |

**CLASSEMENT DE LA DEMANDE (Int. Cl.³)**

A 61 B 17/38
A 61 N 1/06
A 61 N 1/40
A 61 N 1/08

**DOMAINES TECHNIQUES RECHERCHES (Int. Cl.³)**

A 61 N 3/06
A 61 B 17/36
      17/40
      17/39
      17/38
A 61 N 5/00
      1/00
      1/08
      1/06
      1/40
      3/02

**CATEGORIE DES DOCUMENTS CITES**

X: particulièrement pertinent
A: arrière-plan technologique
O: divulgation non-écrite
P: document intercalaire
T: théorie ou principe à la base de l'invention
E: demande faisant interférence
D: document cité dans la demande
L: document cité pour d'autres raisons

&: membre de la même famille, document correspondant

Le présent rapport de recherche a été établi pour toutes les revendications

| Lieu de la recherche | Date d'achèvement de la recherche | Examinateur |
|---|---|---|
| La Haye | 15.05.1981 | BIJN |

OEB Form 1503.1   06.78

| | DOCUMENTS CONSIDERES COMME PERTINENTS | | CLASSEMENT DE LA DEMANDE (Int. Cl. ³) |
|---|---|---|---|
| Catégorie | Citation du document avec indication, en cas de besoin, des parties pertinentes | Revendica-tion concernée | |
| A | <u>FR - A - 858 734</u> (EMYRADIO)<br>* page 1, lignes 42 à 37; page 3, lignes 7 à 9; figure 3 * | 1 | |
| | | | **DOMAINES TECHNIQUES RECHERCHES (Int. Cl. ³)** |

OEB Form 1503.2  06.78